Europäisches Patentamt

(19) European Patent Office

Office européen des brevets

(11) Numéro de publication: **0 069 683**
**B1**

(12) **FASCICULE DE BREVET EUROPEEN**

(45) Date de publication du fascicule du brevet:
17.04.85

(51) Int. Cl.⁴: **A 61 F 2/38**

(21) Numéro de dépôt: **82420069.5**

(22) Date de dépôt: **02.06.82**

(54) **Prothèse totale du genou.**

(30) Priorité: **06.07.81 FR 8113723**

(43) Date de publication de la demande:
**12.01.83 Bulletin 83/2**

(45) Mention de la délivrance du brevet:
**17.04.85 Bulletin 85/16**

(84) Etats contractants désignés:
**AT BE CH DE GB IT LI LU NL SE**

(56) Documents cités:
**DE - A - 2 122 390**
**DE - A - 2 531 080**
**DE - B - 2 549 819**
**US - A - 3 813 700**
**US - A - 4 219 893**

(73) Titulaire: **Rambert, André, Les Fontanelles 10 bis, rue du Docteur Bonhomme, F-69003 Lyon (FR)**
Titulaire: **Bousquet, Gilles, Chemin de Marandon, F-42100 Saint-Etienne (FR)**

(72) Inventeur: **Rambert, André, Les Fontanelles 10 bis, rue du Docteur Bonhomme, F-69003 Lyon (FR)**
Inventeur: **Bousquet, Gilles, Chemin de Marandon, F-42100 Saint-Etienne (FR)**

(74) Mandataire: **Maureau, Pierre, Cabinet GERMAIN & MAUREAU Le Britannia - Tour C 20, Boulevard E. Déruelle, F-69003 Lyon (FR)**

## Description

La présente invention concerne une prothèse totale du genou.

Il existe actuellement deux types de prothèse du genou, à savoir, les prothèses partielles destinées à la restauration d'un seul condyle et les prothèses totales destinées à la restauration des deux condyles.

Dans ce dernier type de prothèse, on distingue des prothèses sans ciment et sans pivot central, et des prothèse scellées avec pivot central

D'une manière générale, les prothèses destinées à la restauration des deux condyles, et quel que soit le type choisi, présentent de nombreux inconvénients tels que résection importante des os, liaison avec l'os par ciment et absence de carter pour l'appui de la rotule. En outre, elles imposent le sacrifice du pivot central sans présenter en compensation des moyens de lutte contre la laxité antéropostérieure et ne permettent pas les rotations internes et externes. Enfin, elles ne permettent généralement pas non plus le changement, d'une manière simple, des surfaces de frottement usées.

C'est ainsi, par exemple, que l'on connaît, par la demande de brevet allemand 2 531 080, une prothèse totale du genou comportant deux éléments dont chacun est destiné à être scellé dans le fémur ou dans le tibia, au niveau de l'articulation du genou, dont l'un présente un bossage cylindro-sphérique et l'autre un logement complémentaire avec ouverture postérieure pour permettre la flexion de la jambe. Il faut noter que, d'une part, chacun des éléments de cette prothèse est destiné à être engagé à force et scellé, et non vissé, dans l'extrémité de l'os correspondant malgré qu'ils soient pourvus de gorges annulaires, en forme de filetage, ce qui n'élimine pas le risque de descellement et que, d'autre part, la liaison ainsi obtenue entre le tibia et le fémur n'est pas suffisamment guidée pour donner satisfaction.

On connaît aussi, par la demande de brevet allemand 2 549 819, une prothèse totale du genou dont chaque élément fémoral ou tibial est muni d'une tige annelée destinée à être engagée à force et scellée dans l'os correspondant. Il faut en ourtre noter que le plateau tibial de cette prothèse est conformé au profil des condyles fémoraux ne procurant aucune liberté autre que la rotation correspondant à la flexion du genou et n'autorisant donc aucune rotation interne et externe.

La présente invention vise à remédier à ces inconvénients. A cet effet, la prothèse qu'elle concerne est du type comportant un élément tibial et un élément fémoral dont chacun est solidaire d'une tige d'ancrage destinée à être fixée dans le canal médullaire de l'os correspondant.

Dans cette prothèse, d'une part chaque tige d'ancrage porte un filetage extérieur conique permettant son vissage dans le canal médullaire du tibia ou du fémur, des moyens étant prévu pour permettre son entraînement en rotation, en vue de ce vissage et, d'autre part, chaque élément tibial ou fémoral est fixé de manière amovible à la tige d'ancrage lui correspondant, chaque tige d'ancrage présentant, à son extrémité extérieure, c'est-à-dire celle à laquelle doit être fixé l'élément fémoral ou tibial, un prolongement cylindrique présentant une portée cylindrique apte à être engagée, à frottement doux, dans un puits de même diamètre ménagé à cet effet dans l'élément considéré, et une extrémité libre filetée apte à recevoir un écrou et éventuellement un contre-écrou de fixation logés dans un prolongement du puits précité, de plus grand diamètre que lui et débouchant dans la face opposée de l'élément considéré, tandis que par ailleurs l'élément fémoral comprend, d'une part, en arrière d'un bossage en saillie sur sa face d'appui et entourant son puits de fixation, deux prolongements postérieurs en arc de cercle, de section sensiblement en cornière et destinés à jouer le rôle de portées condyliennes, ces deux portées condyliennes étant reliées, à l'arrière, par un axe tenu par les ailes latérales de renfort des portées condyliennes qui sont, en outre, destinées à prendre appui sur les parties réséquées des condyles et, d'autre part, en avant du bossage précité, un prolongement unique en forme de bouclier incurvé du côté du bossage et destiné à servir d'organe d'appui et de guidage à la rotule ou, éventuellement, au disque rotulien, et l'élément tibial est constitué par une platine destinée à prendre appui sur la partie réséquée du tibia et présentant, sur sa face d'appui, un bossage entourant son puits de fixation et, sur sa face opposée, d'une part, disposés de part et d'autre du puits de fixation, deux évidements cylindriques dont chacun est destiné à loger le pivot cylindrique de même diamètre que le pivot d'une plateau tibial amovible en une matière présentant un faible coefficient de frottement par rapport à la matière constitutive des portées condyliennes de l'élément fémorale et, d'autre part, décalé vers l'arrière par rapport au puits de fixation, une saillie en forme de chape ouverte dont la lumière est destinée à être engagée sur l'axe reliant les ailes latérales de renfort des portées condyliennes de l'élément fémoral, la différence d'épaisseur entre les plateaux tibiaux déterminant la valeur du valgus physiologique.

La portée cylindrique assure une bonne liaison mécanique entre l'élément considéré et la tige d'ancrage associée. De plus, cette disposition permet un positionnement angulaire précis de chaque élément après mise en place de la tige d'ancrage. Elle permet aussi le retrait d'un élément fémoral ou tibial, sans exiger le retrait de sa tige d'ancrage. Par ailleurs, l'engagement de la chape sur l'axe de l'élément fémoral permet de lutter efficacement contre la laxité antéro-postérieure. Toutefois, pour que cet engagement ne s'oppose pas aux rotations physiologique internes et externes du genou, suivant une caractéristique intéressante de cette prothèse, les parois longitudinales de la lumière de la chape sont taillées en biseau.

Pour améliorer leur tenue à l'extrémité réséquée de l'os considéré, chaque élément fémoral ou tibial présente, sur sa face d'appui, au moins un pion d'ancrage.

Suivant une forme d'exécution avantageuse de l'invention, chaque élément tibial et fémoral est en un métal supportable par l'organisme, tel qu'en acier inoxydable, et chaque plateau tibial est en polyéthylène haute densité.

Pour permettre une tenue acceptable des tiges d'ancrage de chaque élément fémoral ou tibial dans le canal médullaire de l'os correspondant, chaque filetage conique est revêtu d'une céramique d'aluminium tel que l'oxyde d'aluminium, favorable à la corticalisation de l'os spongieux.

Enfin, suivant une autre caractéristique de l'invention, dans le cas où la rotule est défectueuse, il est prévu un disque rotulien apte à prendre appui sur le bouclier prévu à cet effet dans l'élément fémoral.

De toute façon, l'invention sera bien comprise à l'aide de la description qui suit, en référence au dessin schématique annexé représentant, à titre d'exemple non limitatif, une forme d'exécution de cette prothèse:

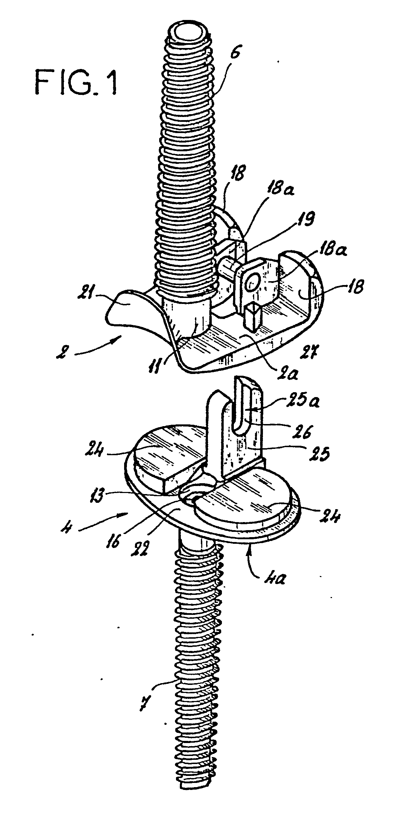

Figure 1 en est une vue en perspective éclatée;

Figure 2 en est une vue de face en élévation;

Figure 3 en est une vue en coupe suivant la ligne III-III de figure 2.

Comme le montre le dessin, cette prothèse est du type comportant un élément fémoral (2) destiné à être fixé à l'extrémité inférieure réséquée d'un fémur (3), et un élément tibial (4) destiné à être fixé à l'extrémité réséquée d'un tibia (5), la fixation de chacun de ces deux éléments (2) et (4) étant assurée par une tige d'ancrage respectivement (6) et (7).

Chaque tige d'ancrage (6) et (7) porte extérieurement un filetage conique permettant son vissage dans le canal médullaire de l'os (3) ou (5) correspondant. Pour permettre une bonne tenue de chaque tige d'ancrage (6) et (7) après sa mise en place, avantageusement, son filet conique est revêtu d'une céramique d'aluminium tel que l'oxyde d'aluminium qui favorise la corticalisation de l'os spongieux.

Comme le montre le dessin, chaque élément fémoral (2) ou tibial (4) est fixé de manière amovible à la tige d'ancrage (6) ou (7) lui correspondant.

Dans l'exemple illustré sur le dessin, chaque tige d'ancrage (6) ou (7) présente, à son extrémité de fixation, c'est-à-dire à celle à laquelle doit être fixé l'élément fémoral (2) ou tibial (4), une portée cylindrique (8) prolongée, à son extrémité libre, par une tige filetée (9) et, chaque élément fémoral (2) ou tibial (4) présente, en saillie sur sa face d'appui respectivement (2a) et (4a) un bossage (11) entourant un puits (12) de même diamètre que la portée cylindrique (8) et présentant un prolongement (13) séparé du puits (12) par un épaulement (14) débouchant dans la face opposée de l'élément considéré et destiné à servir de logement à un écrou (15) et un contre-écrou (16) assurant la fixation de chaque élément (2) ou (4) à sa tige d'ancrage associée (6) ou (7).

Dans l'exemple illustré sur le dessin, les moyens pour permettre l'entraînement en rotation de chaque tige d'ancrage (6) et (7) sont constitués par au moins deux méplats (17) ménagés en des zones diamétralement opposées au voisinage de l'extrémité libre de la portée cylindrique (8) correspondante.

On conçoit qu'avec cette disposition, non seulement il est facile de mettre en place chaque tige d'ancrage (6) et (7) et chaque élément fémoral (2) ou tibial (4) avec le positionnement angulaire désiré, mais, en outre, si nécessaire, chaque élément (2) ou (4) peut être facilement retiré sans qu'il soit nécessaire de retirer sa tige d'ancrage. On peut noter toutefois qu'étant donné la conservation de l'os, une prothèse suppurée pourra être retirée et replacée quelques mois plus tard après traitement.

Comme on peut le constater, la portée cylindrique (8) de chaque tige d'ancrage (6) ou (7) engagée à frottement doux dans le puits (12) de l'élément correspondant, assure une très bonne liaison mécanique à l'assemblage de l'élément (2) ou (4) à sa tige d'ancrage (6) ou (7) et évite que les efforts mécaniques soient transmis à la tige filetée (9) de section plus réduite puisqu'elle est destinée à recevoir les écrous (15) et contre-écrous (16) nécessaires à cette fixation.

Comme le montre le dessin, l'élément fémoral (2) comprend, en arrière du bossage (11) entourant le puits (12) et son prolongement (13), deux prolongements postérieurs (18) en arc de cercle, de section sensiblement en cornière et destinés à jouer le rôle de portée condylienne en remplacement des deux condyles réséqués du fémur (3). Ces deux portées condyliennes (18) sont reliées à l'arrière par un axe (19) tenu par les ailes latérales (18a) des portées condyliennes (18) qui sont, en outre, destinées à prendre appui sur les parties réséquées des condyles du fémur (3).

Cet élément fémoral (2) comprend, en outre, en avant du bossage (11), un prolongement antérieur unique (21) en forme de bouclier, incurvé du côté du bossage (11) et destiné à servir d'organe d'appui et de guidage de la rotule ou éventuellement au disque rotulien prévu en remplacement de celle-ci lorsqu'elle est détériorée.

L'élément tibial (4) correspondant à cet élément fémoral (2) est constitué par une platine (22) dont la face d'appui (4a) porte le bossage (11) précité et qui présente, débouchant sur sa face opposée, deux évidements cylindriques (23) disposés de part et d'autre du prolongement (13) de son puits de fixation (12), chaque évidement cylindrique (23) étant destiné à loger le pivot (24a) de même diamètre que lui d'un plateau tibial (24) amovible, en une matière présentant un faible coefficient de frottement par rapport à la matière constitutive des portées condyliennes (18) de l'élément fémoral (2).

Dans le cas où l'élément fémoral et ses portées condyliennes (18) sont en acier inoxydable, une matière convenant parfaitement à la réalisation de chaque plateau tibial (24) est le polyéthylène haute densité.

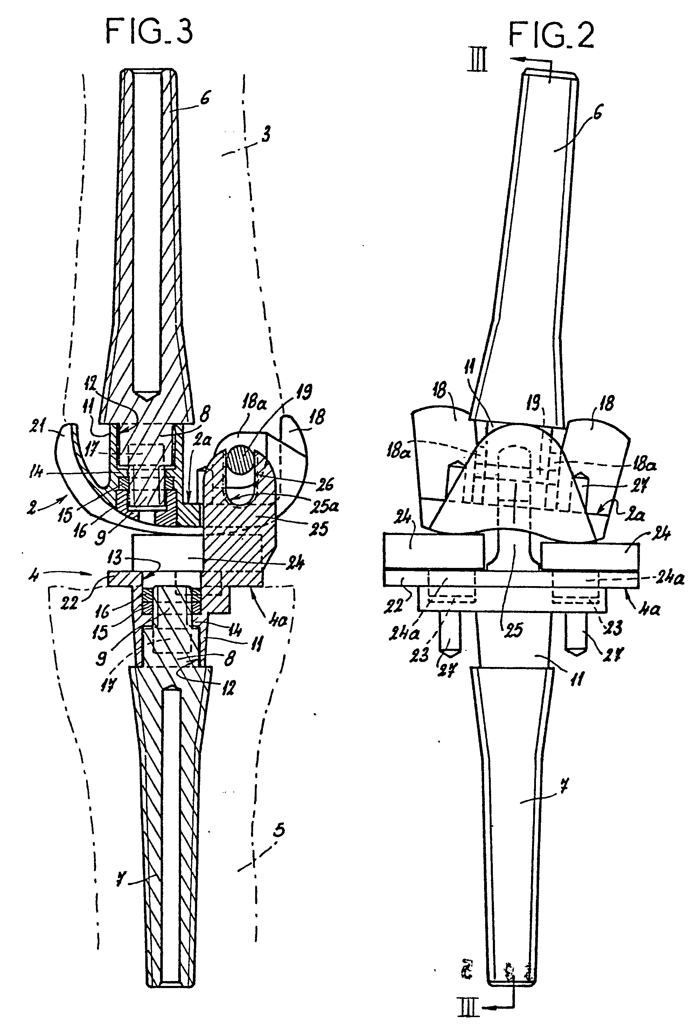

Comme le montre notamment la figure 2, le valgus physiologique est obtenu par un choix judicieux de la différence d'épaisseur des deux plateaux tibiaux (24) utilisés.

L'élément tibial (4) présente, en outre, décalée vers l'arrière par rapport au puits de fixation (12) et à son prolongement (13) une saillie (25) en forme de chape ouverte et dont la lumière (25a) est destinée à être engagée sur l'axe (19) reliant les portées condyliennes (18).

Cet engagement a pour effet de lutter efficacement contre la laxité antéro-postérieure du genou.

Toutefois, pour que cet engagement ne nuise pas aux rotations physiologiques internes et externes du genou, les parois longitudinales opposées de la lumière (25a) sont biseautées, comme indiqué en (26) sur la figure 3.

Dans l'exemple illustré sur le dessin, chaque face d'appui (2a) et (4a) de chaque élément fémoral (2) et tibial (4), porte deux pions d'ancrage (27) destinés à améliorer la tenue de l'élément considéré par pénétration dans l'extrémité réséquée de l'os correspondant.

Comme on le voit, les pièces soumises à usure de cette prothèse et qui sont essentiellement chaque plateau tibial (24), peuvent être facilement retirées et remplacées par une pièce neuve sans que cela nécessite une intervention chirurgicale très importante.

Par rapport aux prothèses du genou actuellement connues, la prothèse selon l'invention présente donc l'avantage d'une grande simplicité de mise en place, d'une grande souplesse d'utilisation et d'une grande facilité de maintien en bon état de fonctionnement.

## Revendications

1. Prothèse totale du genou du type comportant un élément fémoral (2) et un élément tibial (4) dont chacun est solidaire d'une tige d'ancrage (6, 7) destinée à être fixée dans le canal médullaire de l'os correspondant (3, 5), chaque tige d'ancrage (6, 7) portant un filetage extérieur conique et au moins un desdits éléments fémoral (2) ou tibial (4) étant fixé, de manière amovible, à la tige d'ancrage (6, 7) lui correspondant laquelle présente, à son extrémité extérieure, c'est-à-dire celle à laquelle doit être fixé l'élément fémoral (2) ou tibial (4), un prolongement présentant une portée (8) apte à être engagée, dans un puits (12) de forme complémentaire ménagé, à cet effet, dans l'élément (2, 4) considéré, caractérisé en ce que chaque tige d'ancrage (6, 7) comporte des moyens d'entrainement en rotation en vue de son vissage dans l'os correspondant, en ce que lesdits éléments (2, 4) sont tous deux fixés de manière amovible à leurs tiges d'ancrage (6,7), en ce que celles-ci comportent chacune un prolongement dont la portée (8) est cylindrique et s'engage à frottement doux dans le puits (12) et une extrémité libre filetée (9) apte à recevoir un écrou (15) et éventuellement un contre-écrou (16) de fixation logé dans un prolongement (13) du puits (12) précité de plus grand diamètre que lui, dont il est séparé par un épaulement (14) et qui débouche dans la face opposée de l'élément (2,4) considéré, en ce que l'élément fémoral (2), comprend, d'une part, en arrière d'un bossage (11) en saillie sur sa face d'appui (2a) et entourant son puits de fixation (12), deux prolongements postérieurs (18) en arc de cercle, de section sensiblement en cornière et destinés à jouer le rôle de portées condyliennes, ces deux portées condyliennes (18) étant reliées, à l'arrière, par un axe (19) tenu par les ailes latérales (18a) de renfort des portées condyliennes (18) qui sont, en outre, destinées à prendre appui sur les parties réséquées des condyles et, d'autre part, en avant du bossage (11), un prolongement unique (21) en forme de bouclier incurvé du côte du bossage (11) et destiné à servir d'organe d'appui et de guidage à la rotule ou, éventuellement, au disque rotulien, et en ce que l'élément tibial (4) est constitué par une platine (22) destinée à prendre appui sur la partie réséquée du tibia (5) et présentant, sur sa face d'appui (4a), un bossage (11) entourant son puits de fixation (12) et, sur sa face opposée, d'une part, disposés de part et d'autre du puits de fixation (12), deux évidements cylindriques dont chacun est destiné à loger le pivot cylindrique (23) de même diamètre que le pivot (24a) d'un plateau tibial (24) amovible en une matière présentant un faible coefficient de frottement par rapport à la matière constitutive des portées condyliennes (18) de l'élément fémoral (2) et, d'autre part, décalé vers l'arrière par rapport au puits de fixation (12), une saillie (25) en forme de chape ouverte dont la lumière (25a) est destinée à être engagée sur l'axe (19) reliant les ailes latérales (18a) de renfort des portées condyliennes (18) de l'élément fémoral (2), la différence d'épaisseur entre les plateaux tibiaux (24) déterminant la valeur du valgus physiologique.

2. Prothèse totale du genou selon la revendication 1, caractérisée en ce que les parois longitudinales de la lumière (25a) de la chape (25) sont taillées en biseau.

3. Prothèse totale du genou selon l'une quelconque des revendications précédentes, caractérisée en ce que chaque élément fémoral (2) ou tibial (4) présente, sur sa face d'appui (2a, 4a) au moins un pion d'ancrage (27).

4. Prothèse totale du genou selon l'une quelconque des revendications précédentes, caractérisée en ce que chaque élément fémoral (2) et tibial (4) est en un métal supportable par l'organisme, tel qu'en acier inoxydable.

5. Prothèse totale du genou selon la revendication 4, caractérisée en ce que chaque plateau tibial (24) est en polyéthylène haute densité.

6. Prothèse totale du genou selon l'une quelconque des revendications précédentes, caractérisée en ce que le filetage conique de chaque tige d'ancrage (6, 7) est revêtu d'une céramique d'aluminium tel que l'oxyde d'aluminium, favorable à la corticalisation de l'os spongieux.

7. Prothèse totale du genou selon l'une quelconque des revendications précédentes, caractérisée en ce que, dans le cas où la rotule est défectueuse, il est prévu un dique rotulien apte à prendre appui sur le bouclier (21) prévu à cet effet dans l'élément fémoral (2).

**Patentansprüche**

1. Knievollprothese des Typs mit einem femoralen Element (2) und einem tibialen Element (4), von denen jedes fest mit einer Verankerungsstange (6,7) verbunden ist, die dazu bestimmt sind, in dem Markkanal des entsprechenden Knochens (3, 5) befestigt zu werden, wobei jede Verankerungsstange (6, 7) ein konisches Außengewinde trägt und mindestens eines der genannten femoralen (2) oder tibialen (4) Elemente lösbar an der ihm entsprechenden Verankerungsstange (6, 7) befestigt ist, welch letztere an ihrem Außenende, d. h. an demjenigen, an dem das femorale (2) oder tibiale (4) Element befestigt werden muß, eine Verlängerung mit einem Abschnitt (8) aufweist, der in eine Bohrung (12) komplementärer Form einsteckbar ist, die zu diesem Zweck im betroffenen Element (2, 4) vorgesehen ist, dadurch gekennzeichnet, daß jede Verankerungsstange (6, 7) Mittel zu einem drehenden Antreiben im Hinblick auf ihre Einschraubung in den entsprechenden Knochen aufweist, daß die genannten Elemente (2, 4) lösbar an ihren Verankerungsstangen (6, 7) befestigt sind, daß sie ferner beide eine Verlängerung haben, deren Abschnitt (8) zylindrisch ist und mit leichter Reibung in die Bohrung (12) einsteckbar ist und ein freies mit Gewinde versehenes Ende (9) aufweist, auf das eine Mutter (15) und eventuell eine Kontermutter (16) für die Befestigung aufschraubbar ist und dies in einer Verlängerung (13) der genannten Bohrung (12), mit größerem Durchmesser als diese, und von dieser durch eine Schulter (14) getrennt, welche Verlängerung (13) auf der entgegengesetzten Fläche des betroffenen Elementes (2, 4) mündet, daß ferner das femorale Element (2) einerseits rückwärtig eines über seine Stützfläche (2a) vorstehenden und die Befestigungsbohrung (12) umgebenden Vorsprunges (11) zwei rückwärtige, kreisbogenförmige Verlängerungen (18) aufweist, die einen im wesentlichen eckigen Querschnitt haben und dazu bestimmt sind, die Rolle von Kondylenbereichen zu spielen, wobei diese beiden Kondylenbereiche (18) rückwärtig durch eine Achse (19) verbunden sind, die durch seitliche Verstärkungsstege (18a) der Kondylenbereiche (18) gehalten ist, welch letztere darüberhinaus dazu bestimmt sind, sich auf den resizierten Abschnitt der Kondylen abzustützen, und andererseits vor dem Vorsprung (11) eine einzelne Verlängerung (21) in Form eines Schildes vorgesehen ist, die zur Seite des Vorsprunges (11) gebogen ist und dazu bestimmt ist, als Abstütz- und Führungsorgan für die Kniescheibe oder eventuell einer die Kniescheibe ersetzenden Scheibe zu dienen, und daß ferner das tibiale Element (4) aus einer Platine (22) gebildet ist, die dazu bestimmt ist, sich auf dem resizierten Bereich des Schienbeines (5) abzustützen und die auf ihrer Abstützfläche (4a) einen seine Befestigungsbohrung (12) umgebenden Vorsprung (11) aufweist, sowie auf seiner gegenüberliegenden Fläche einerseits, zu beiden Seiten der Befestigungsbohrung (12) angeordnet, zwei zylindrische Ausnehmungen, die beide dazu bestimmt sind, den zylindrischen Zapfen (24a) gleichen Durchmessers wie sie selbst einer lösbar angeordneten tibialen Platte (24) aufzunehmen, die aus einem Material mit geringem Reibkoeffizienten bezüglich des die Kondylenbereiche (18) des femoralen Elementes (2) bildenden Materiales besteht und andererseits, nach hinten bezüglich der Befestigungsbohrungen (12) versetzt, ein Vorsprung (25) in Form eines offenen Gabelstückes vorgesehen ist, dessen Schlitz (25a) dazu bestimmt ist, auf die die seitlichen Verstärkungsstege (18a) der Kondylenbereiche (18) des femoralen Elementes (2) verbindenden Achse (19) aufgesetzt zu werden, wobei die Dickendifferenz zwischen den tibialen Platten (24) die Größe des physiologischen Valgus bestimmt.

2. Knievollprothese nach Anspruch 1, dadurch gekennzeichnet, daß die Längswände des Schlitzes (25a) des Gabelstückes (25) mit scharfer Kante ausgebildet sind.

3. Knievollprothese nach einem der vorhergehenden Ansprüche, dadurch gekennzeichnet, daß jedes femorale (2) oder tibiale (4) Element auf seiner Abstützfläche (2a, 4a) mindestens einen Verankerungspion (27) aufweist.

4. Knievollprothese nach einem der vorhergehenden Ansprüche, dadurch gekennzeichnet, daß jedes femorale (2) und tibiale (4) Element aus einem für den Organismus verträglichen Metall, wie beispielsweise aus nichtoxidierendem Stahl, besteht.

5. Knievollprothese nach Anspruch 4, dadurch gekennzeichnet, daß eine jede tibiale Platte (24) aus Polyäthylen hoher Dichte besteht.

6. Knievollprothese nach einem der vorhergehenden Ansprüche, dadurch gekennzeichnet, daß das konische Gewinde einer jeden Verankerungsstange (6, 7) mit einem keramischen Material auf Aluminiumbasis, wie Aluminiumoxyd, verkleidet ist, das die Kortikalisation des Schwammbereiches des Knochens begünstigt.

7. Knievollprothese nach einem der vorhergehenden Ansprüche, dadurch gekennzeichnet, daß im Falle einer defekten Kniescheibe eine diese ersetzende Scheibe vorgesehen ist, die an dem Schild (21) abstützbar ist, der zu diesem Zweck an dem femoralen Element (2) vorgesehen ist.

## Claims

1. A complete prosthesis of the knee, of the type comprising a femoral unit (2) and a tibial unit (4) each one of which is rigidly fitted to an anchoring stem (6, 7) intended to be fixed into the medullary canal of the corresponding bone (3, 5), each anchoring stem (6, 7) bearing an external conical thread and at least one of the said femoral (2) or tibial (4) units being fitted in detachable manner to the anchoring stem (6, 7) corresponding to it, which stem presents at its external extremity, that is to say at the extremity to which the femoral (2) or tibial (4) unit is to be fitted, an extension featuring a boss (8) suitable to be engaged into a bored hole (12) of complementary shape arranged to this end in the unit (2, 4) in question, characterized in that each anchoring stem (6, 7) comprises means of causing rotation for the purpose of screwing it into the corresponding bone, and that both the said units (2, 4) are fitted in removable manner to their anchoring stem (6, 7); in that each of these stems comprises an extension the boss (8) of which is cylindrical and engages as an easy fit into the bored hole (12), and a threaded free end (9) suitable to receive a nut (15) and possibly a fixing lock-nut (16) accommodated in an extension (13) of the above-mentioned bored hole (12) but of greater diameter, separated from the bored hole (12) by a shoulder (14) and emergiong in the opposite face of the unit (2, 4) in question; in that the femoral unit (2) comprises on one hand behind a boss (11), projecting from its supporting surface (2a) and surrounding its fixing hole (12), two rear extensions (18) as an arc of a circle, of more or less valley-shaped section and intended to play the part of the condyle bosses, these two condyle bosses (18) being connected together at the rear by a shaft (19) held by the two side reinforcement wings (18a) of the condyle bosses (18) which are additionally intended to bear against the resected portions of the condyles, and on the other hand in front of boss (11) a single extension (21) in the form of a shield inwardly curved towards boss (11) and intended to serve as a component for application and guidance of the patella or possibly of the patella disc; and in that the tibial unit (4) is formed by a plate (22) intended to rest against the resected portion of the tibia (5) and featuring, on its supporting surface (4a) a boss (11) surrounding its fixing hole (12) and, on its opposite surface, on one hand two cylindrical cavities, one on each side of the fixing hole (12), each of which is intended to accommodate the cylindrical pivot (23) of the same diameter as the pivot (24a) of a removable tibial platform (24) of material having a low coefficient of friction against the material forming the condyle bosses (18) of the femoral unit (2) and, on the other hand, displaced towards the rear with reference to the fixing hole (12), a projection (25) in the form of an open fork, the aperture (25a) of which is intended to engage the shaft (19) connecting together the side reinforcement wings (18a) of the condyle bosses (18) of the femoral unit (2), the difference of thickness between the two tibial platforms (24) determining the degree of physiological valgus.

2. A complete prosthesis of the knee according to Claim 1, characterized in that the longitudinal walls of the aperture (25a) of the form (25) are bevel-cut.

3. A complete prosthesis of the knee according to one of the preceding Claims, characterized in that each femoral (2) or tibial (4) unit presents, on its supporting surface (2a, 4a), at least one anchoring stud (27).

4. A complete prosthesis of the knee according to any of the preceding Claims, characterized in that each femoral (2) and tibial (4) unit is of a metal tolerated by the body system, such as stainless steel.

5. A complete prosthesis of the knee according to Claim 4, characterized in that each tibial platform (25) is of high density polyethylene.

6. A complete prosthesis of the knee according to any of the preceding Claims, characterized in that each anchoring stem (6, 7) is coated with an aluminium ceramic such as aluminium oxide, favourable to corticalization of the spongy bone.

7. A complete prosthesis of the knee according to any of the preceding Claims, characterized in that in cases where the patella is defective a patella disc is provided, suitable to support itself against the shield (21) provided in the femoral unit (2) for this purpose.

FIG.1

0 069 683

FIG.3    FIG.2

9